(19) Europäisches Patentamt
European Patent Office
Office européen des brevets

(11) Veröffentlichungsnummer: **0 198 370 B1**

(12) **EUROPÄISCHE PATENTSCHRIFT**

(45) Veröffentlichungstag der Patentschrift: **13.05.92**

(51) Int. Cl.$^5$: **C07C 271/04**

(21) Anmeldenummer: **86104678.7**

(22) Anmeldetag: **05.04.86**

# (54) Verfahren zur Herstellung von Carbamidsäurechloriden sekundärer Amine.

(30) Priorität: **16.04.85 DE 3513599**

(43) Veröffentlichungstag der Anmeldung:
**22.10.86 Patentblatt 86/43**

(45) Bekanntmachung des Hinweises auf die Patenterteilung:
**13.05.92 Patentblatt 92/20**

(84) Benannte Vertragsstaaten:
**BE CH DE FR GB IT LI NL**

(56) Entgegenhaltungen:
**DE-A- 2 206 365**

(73) Patentinhaber: **HOECHST AKTIENGESELLSCHAFT**
**Postfach 80 03 20**
**W-6230 Frankfurt am Main 80(DE)**

(72) Erfinder: **Semler, Günther, Dr.**
**Johann-Strauss-Strasse 44**
**W-6233 Kelkheim (Taunus)(DE)**
Erfinder: **Schaeffer, George, Dr.**
**Herderstrasse 58**
**W-6238 Hofheim am Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.08/2.19/2.0)

## Beschreibung

Carbamidsäurechloride sekundärer Amine sind Verbindungen der Formel

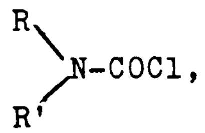

worin R und R' organische Reste bedeuten.

Sie sind hauptsächlich Zwischenprodukte auf verschiedenen Sachgebieten, insbesondere auf dem Pflanzenschutzsektor.

Die am häufigsten angewandte Methode zur Herstellung von Carbamidsäurechloriden besteht in der Umsetzung von Aminen mit Phosgen; vgl. Houben-Weyl, Methoden der Organischen Chemie, Bd. E4 "Kohlensäure-Derivate" (1983) S. 45. Die Umsetzung sekundärer Amine läßt sich durch folgende Reaktionsgleichung - z.B. mit Methylamin als Ausgangs-Amin - wiedergeben:

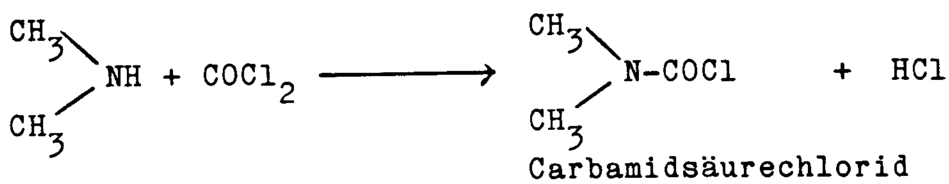

Carbamidsäurechlorid

Das Carbamidsäurechlorid reagiert mit dem Ausgangs-Amin sehr leicht zum entsprechenden Harnstoffderivat weiter:

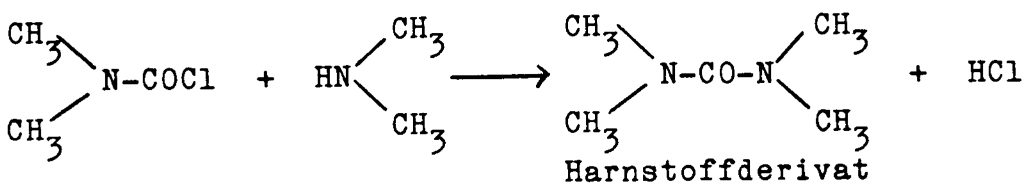

Harnstoffderivat

Falls man hohe Ausbeuten und Reinheiten an Carbamidsäurechlorid erzielen will, müssen daher Maßnahmen zur Vermeidung der Weiterreaktion des Carbamidsäurechlorids zum Harnstoffderivat getroffen werden. Die Bildung derartiger Harnstoffderivate aus Aminen und Phosgen wird beispielsweise in der DE-A 22 06 366 beschrieben.

Speziell zur Herstellung von Carbamidsäurechloride sekundärer Amine wird deswegen auf Seite 47 des vorerwähnten Houben-Weyl-Bandes empfohlen, Phosgen bei niedriger Temperatur in eine Lösung des Amins in einem indifferenten Lösungsmittel einzuleiten. Dabei wird die Hälfte des Amins in das Hydrochlorid überführt. Aus diesem Reaktionsgemisch kann man das Carbamidsäurechlorid isolieren. Vorteilhafterweise jedoch erhitzt man das Gemisch unter weiterem Durchleiten von Phosgen auf über 100 °C, wobei das Amin-Hydrochlorid in das Carbamidsäurechlorid übergeht.

Im Anschluß hieran heißt es in dieser Houben-Weyl-Stelle dann zwar, daß man die Phosgenierung auch häufig gleich in der Hitze durchführen kann; konkrete Beispiele für die Herstellung von Carbamidsäurechloriden sekundärer Amine durch Einleiten von Phosgen in das vorgelegte Amin bei erhöhten Temperaturen sind jedoch nur mit solchen sekundären Aminen beschrieben, bei denen die Reaktionsfähigkeit durch mindestens einen aromatischen Rest stark herabgesetzt ist. Wendet man diese Verfahrensweise auf sekundäre rein aliphatische Amine, wie z.B. N,N-Di-n-propylamin oder N,N-Di-n-butylamin, an, dann erhält man infolge deren hoher Reaktivität - wie eigene Versuche gezeigt haben - neben den durch Weiterreaktion entstandenen Harnstoffderivaten und deren Umsetzungsproduktenmit Phosgen (wobei sich Amidchloride bilden, die unter den angewandten Temperaturen instabil sind und Zersetzungsprodukte bilden) das

gewünschte Carbamidsäurechlorid in nur mäßiger Reinheit und Ausbeuten zwischen 60 und 80 %. Dieses Ergebnis läßt die Anwendung der vorerwähnten Methode auf die Herstellung von Carbamidsäurechloriden sekundärer aliphatischer Amine, insbesondere in technischem Maßstab, kaum als geeignet erscheinen.

Überraschenderweise wurde nun jedoch gefunden, daß die Methode dann sehr gut funktioniert - d.h. Ausbeuten an Carbamidsäurechloriden von z.T. erheblich über 90 % d. Th. bei hohen Produktreinheiten liefert -, wenn man von solchen sekundären aliphatischen Aminen ausgeht, deren Alkylgruppen in der 1-Stellung verzweigt sind.

Erfindungsgegenstand ist daher ein Verfahren zur Herstellung von Carbamidsäurechloriden sekundärer Amine durch Einleiten von Phosgen in die vorgelegten - gegebenenfalls in einem inerten Lösungsmittel gelösten - sekundären Amine in flüssiger Phase,
das dadurch gekennzeichnet ist, daß man als sekundäre Amine sekundäre aliphatische Amine mit in der 1-Stellung verzweigten Alkylgruppen der Formel I

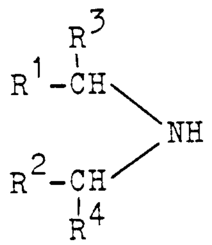

(I),

worin

$R^1$ und $R^2$ = unabhängig voneinander $C_1$-$C_{20}$-Alkyl, vorzugsweise $C_1$-$C_7$-Alkyl,

$R^3$ und $R^4$ = unabhängig voneinander $C_1$-$C_4$-Alkyl, vorzugsweise $C_1$-$C_2$-Alkyl,

oder die Gruppen $R^1$ + $R^3$ und/oder $R^2$ + $R^4$ zusammen = $-(CH_2)_4-$ oder $-(CH_2)_5-$
bedeuten, verwendet, und daß man die Umsetzung bei Temperaturen zwischen etwa 80 und 160 °C durchführt.

Unter die Formel I fallende beispielhafte Ausgangs-Amine sind N,N-Diisopropylamin, N,N-Di-sekund.-butylamin, N,N-Di-(2-methyl-butyl)-amin, N,N-Di-cyclopentylamin, N,N-Dicyclohexylamin, etc.

Besonders bevorzugte Ausgangs-Amine sind N,N-Diisopropylamin, N,N-Di-sek.-butylamin und N,N-Dicyclohexylamin.

Die Ausgangsamine können in bei derartigen Phosgenierungen üblichen indifferenten Lösungsmitteln wie z.B. in aromatischen Kohlenwasserstoffen (z.B. Toluol, Xylol etc.) und Chlorkohlenwasserstoffen (z.B. Chlorbenzol, Dichlorbenzole etc.) gelöst werden. Der Einsatz solcher Lösungsmittel ist jedoch nur in den Fällen erforderlich, in denen durch intermediär entstehende, schwer lösliche Hydrochloride schwer rührbare Reaktionsgemische auftreten.

Speziell im Falle der Verwendung von N,N-Di-sek.-butylamin als Ausgangs-Amin ist es bevorzugt, ohne den Einsatz eines indifferenten Lösungsmittels zu arbeiten.

Die Reaktionstemperatur liegt vorzugsweise zwischen etwa 100 und 130 °C.

Als Reaktionsdruck ist Normaldruck bevorzugt.

Das Verfahren wird so durchgeführt, daß in das auf die gewünschte Reaktionstemperatur gebrachte vorgelegte - gegebenenfalls in einem indifferenten Lösungsmittel gelöste - sekundäre Amin Phosgen eingeleitet wird. Eine genaue Dosierung des Phosgens ist dabei ebenso wenig notwendig wie ein Überschuß. Dadurch lassen sich größere Mengen Phosgen in den Reaktionsgemischen vermeiden, was bei der Giftigkeit dieser Chemikalie von großer Bedeutung ist.

Die Aufarbeitung des Reaktionsansatzes erfolgt auf übliche Weise, vorzugsweise durch Destillation.

Wegen der einfachen Verfahrensweise, der durchweg z.T. erheblich über 90 % d.Th. liegenden Carbamidsäurechlorid-Ausbeuten und der hohen Produktreinheit stellt das Verfahren einen erheblichen Fortschritt auf diesem Gebiet dar.

Beim Einsatz der Ausgangs-Amine der Formel I besitzen die erfindungsgemäß erhaltenen Carbamidsäurechloride die nachstehende Formel II:

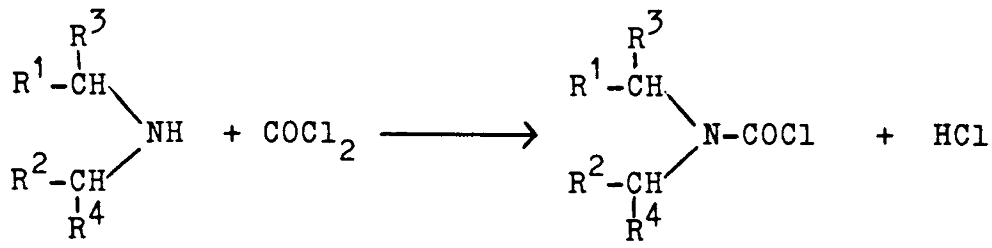

(I) (II)

$R^1$ - $R^4$ besitzen die vorher bei Formel I angegebene Bedeutung.

Die nachstehenden Beispiele sollen der weiteren Erläuterung der Erfindung dienen. Auf die Erfindungsbeispiele (A) folgen noch einige Vergleichsbeispiele (B), welche zeigen, daß das erfindungsgemäße Verfahren mit solchen sekundären aliphatischen Aminen, die in 1-Stellung nicht verzweigt sind, nur in unbefriedigender Weise funktioniert.

A) Erfindungsbeispiele

Beispiel 1

**N,N-Di-sek-butylcarbamidsäurechlorid** $(CH_3-CH_2-\overset{CH_3}{CH})_2N-COCl$
(durch direkte Phosgenierung von N,N-Di-sek-butylamin ohne Lösungsmittel)

129,0 g (= 1 Mol) Di-sek-butylamin wurden bei 110 °C so lange mit Phosgen begast, bis an einem bei -20 °C betriebenen Rückflußkühler Phosgenrückfluß einsetzte. Anschließend wurde noch 2 Stunden bei gleicher Temperatur gehalten. Insgesamt wurden in ca. 5 Stunden etwa 190 g Phosgen eingeleitet, von denen während der Reaktion ein Teil mit dem entstehenden Chlorwasserstoff aus dem Reaktionsgefäß entwich; das restliche überschüssige Phosgen wurde anschließend bei 70 °C mittels $N_2$ ausgeblasen. Es verblieben etwa 190 g (= 99,2 % d.Th.) eines etwa 99 %igen Rohproduktes (GC), das zur Reinigung im Vakuum destilliert wurde. Dabei gingen bei 122 °C/24 mbar 182,0 g eines fast farblosen Produktes über, dessen Reinheit bei 99,6 % lag. Die Ausbeute an Carbamidsäurechlorid 100 %ig betrug damit 181,3 g entsprechend 94,7 % d.Th. Im Destillierkolben verblieb ein Rückstand von 6,5 g (entsprechend 3,4 % der theoretischen Ausbeute).

Beispiel 2

**N,N-Di-sek-butylcarbamidsäurechlorid** $(CH_3-CH_2-\overset{CH_3}{CH})_2N-COCl$
(durch direkte Phosgenierung von N,N-Di-sek-butylamin in Chlorbenzol)

Ein Gemisch aus 129,0 g Di-sek-butylamin und 129,0 g Chlorbenzol wurde entsprechend Beispiel 1 mit Phosgen umgesetzt. Nach beendeter Phosgenierung wurde noch 1 Stunde am Rückfluß erhitzt, anschließend das restliche Phosgen durch Ausblasen mit Stickstoff entfernt und nach dem Abtrennen des Lösungsmittels bei reduziertem Vakuum das Hauptprodukt überdestilliert. Man erhielt auf diese Weise das

N,N-Di-sek-butylcarbamidsäurechlorid in einer Ausbeute von 186,0 g mit einer Reinheit von 99,8 %. Die Ausbeute an Carbamidsäurechlorid 100 %ig betrug 185, 6 g entsprechend 96,9 % d.Th. Im Destillierkolben verblieb ein Rückstand von 2,5 g (entsprechend 1,2 % d.theoretischen Ausbeute).

Beispiel 3

**N,N-Diisopropylcarbamidsäurechlorid** $(CH_3-CH(CH_3))_2N-COCl$
**(durch direkte Phosgenierung von N,N-Diisopropylamin in Chlorbenzol)**

101,0 g (= 1 Mol) Diisopropylamin wurden in 505,0 g Chlorbenzol bei 120 ° C gemäß Beispiel 2 mit Phosgen umgesetzt und aufgearbeitet. Aus einem etwa 99 %igen Reaktionsgemisch (ohne Lösungsmittel gerechnet) wurden 154,5 g eines bei 141 ° C/133 mbar siedenden, etwa 99 %igen Destillates mit einem Erstarrungspunkt von59 ° C erhalten. Die Ausbeute an 100 %igem Carbamidsäurechlorid betrug damit 153,0 g, entsprechend 93,6 % d.Th.

Beispiel 4

**N,N-Di-cyclohexylcarbamidsäurechlorid** ( 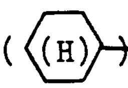 $)_2N-COCl$
**(durch direkte Phosgenierung von N,N-Dicyclohexylamin)**

45,3 g (= 0,25 Mol) Dicyclohexylamin wurden in 100 g o-Dichlorbenzol gelöst und bei 160 ° C so lange mit Phosgen begast, bis keine Phosgenaufnahme mehr zu beobachten war. Nach 2-stündiger Nachreaktion bei gleicher Temperatur wurde das überschüssige Phosgen bei etwa 100 ° C mit $N_2$ ausgeblasen und das Reaktionsgemisch aufdestilliert. Das Dicyclohexylcarbamidsäurechlorid destillierte bei 135 ° C/1,2 mbar als fast farblose, bei 84 ° C erstarrende Flüssigkeit über. Die Ausbeute betrug 57,8 g entsprechend 94,9 % d.Th., die Reinheit praktisch 100 % (Cl-Titration).

Ein ähnliches Ergebnis wurde erhalten, als die Phosgenierung in der 5-fachen Menge Chlorbenzol bei 110 - 120 ° C durchgeführt wurde.

B) Vergleichsbeispiele

Vergleichsbeispiel 1

N,N-Di-n-butyl-carbamidsäurechlorid $(CH_3-CH_2-CH_2-CH_2)_2N-COCl$
(durch direkte Phosgenierung von N,N-Di-n-butylamin in Chlorbenzol)

Als Di-n-butylamin gemäß (Erfindungs-)Beispiel 2 mit Phosgen umgesetzt wurde, wurde schon bald nach Beginn der Phosgendosierung ein sehr dickes, kaum noch rührbares Reaktionsgemisch erhalten.Es wurde später wieder dünnflüssig und zuletzt klar. Es enthielt nach vollständigem Umsatz des Amins neben dem gewünschten Carbamidsäurechlorid große Mengen N,N,N',N'-Tetra-n-butylharstoff (ca. 30 % ohne Lösungsmittel gerechnet), der sich mit Phosgen in langsamer Reaktion weiter umsetzte unter Bildung von Carbamidchloriden und dessen Zersetzungsprodukten. Bei der destillativen Aufarbeitung eines solchen Reaktionsproduktes, das neben ca. 70 % Carbamidsäurechlorid (ohne Lösungsmittel gerechnet) eine große Menge Tief- und Hochsieder enthielt, wurden etwa 147 g eines nur ca. 86 %igen Produktes erhalten. Dies entspricht einer Carbamidsäurechlorid-Ausbeute von nur 66,0 % d.Th.

Eine Phosgenierung ohne die Verwendung eines Lösungsmittels gemäß (Erfindungs-)Beispiel 1 war praktisch nicht möglich, da hierbei ein nicht mehr rührbares Reaktionsgemisch entstand.

Vergleichsbeispiel 2

N,N-Di-isobutylcarbamidsäurechlorid 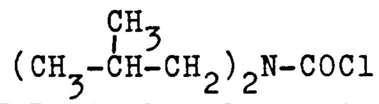
(durch direkte Phosgenierung von N,N-Di-isobutylamin in Chlorbenzol)

Es wurde Diisobutylamin gemäß (Erfindungs-)Beispiel 2 mit Phosgen umgesetzt. Der Verlauf der Phosgenierung war ähnlich wie der von Di-n-butylamin im Vergleichsbeispiel 1. Schließlich wurden etwa 182 g eines nur 70 %igen Produktes, entsprechend einer Carbamidsäurechlorid-Ausbeute von nur 66,5 % d.Th. erhalten.

Vergleichsbeispiel 3

N,N-Di-n-propylcarbamidsäurechlorid $(CH_3-CH_2-CH_2)_2N-COCl$
(durch direkte Phosgenierung von N,N-Di-n-propylamin in Chlorbenzol)

Es wurden Di-n-propylamin wie in (Erfindungs-)Beispiel 3 bei 110 °C mit Phosgen umgesetzt. Dabei entstand ein nur etwa 67 %iges Reaktionsgemisch (ohne Lösungsmittel gerechnet), aus dem durch Destillation etwa 135 g eines ca. 80 %igen Produktes erhalten wurden. Dies entspricht einer Carbamidsäurechlorid-Ausbeute von nur 66,1 % d.Th.

Bei einer Phosgenierungstemperatur von zunächst 5, später 80 °C wurden bei sonst gleicher Arbeitsweise 139 g eines etwa 95 %igen Destillates erhalten. Dies entspricht einer Carbamidsäure-chlorid-Ausbeute von nur 80,8 % d.Th.

**Patentansprüche**

**1.** Verfahren zur Herstellung von Carbamidsäurechloriden sekundärer Amine durch Einleiten von Phosgen in die vorgelegten - gegebenenfalls in einem indifferenten Lösungsmittel gelösten - sekundären Amine in flüssiger Phase, dadurch gekennzeichnet, daß man als sekundäre Amine sekundäre aliphatische Amine mit in der 1-Stellung verzweigten Alkylgruppen der Formel I

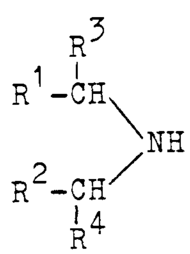
(I),

worin
$R^1$ und $R^2$ = unabhängig voneinander $C_1$-$C_{20}$-Alkyl, vorzugsweise $C_1$-$C_7$-Alkyl,
$R^3$ und $R^4$ = unabhängig voneinander

$C_1$-$C_4$-Alkyl,

vorzugsweise $C_1$-$C_2$-Alkyl,
oder die Gruppen $R^1$ + $R^3$ und/oder $R^2$ + $R^4$ zusammen = $-(CH_2)_4-$ oder $-(CH_2)_5-$
bedeuten, verwendet, und daß man die Umsetzung bei Temperaturen zwischen etwa 80 und 160 °C durchführt.

**2.** Verfahren nach den Ansprüchen 1 bis 2, dadurch gekennzeichnet, daß man als sekundäre aliphatische Amine mit in der 1-Stellung verzweigten Alkylgruppen N,N-Diisopropylamin, N,N-Di-sek.-butylamin oder N,N-Dicyclohexylamin verwendet.

**3.** Verfahren nach den Ansprüchen 1 bis 3, dadurch gekennzeichnet, daß man in Falle der Verwendung von N,N-Di-sek.-butylamin ohne Einsatz eines indifferenten Lösungsmittels arbeitet.

**4.** Verfahren nach den Ansprüchen 1 bis 4, dadurch gekennzeichnet, daß man die Umsetzung bei Temperaturen zwischen etwa 100 und 130 ° C, durchführt.

## Claims

**1.** A process for the preparation of carbamoyl chlorides derived from secondary amines by passing phosgene in liquid phase into the secondary amines which have been initially taken - if appropriate dissolved in an inert solvent - which comprises using, as the secondary amines, secondary aliphatic amines having alkyl groups which are branched in the 1-position of the formula I

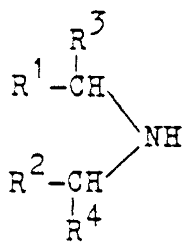

(I),

in which

$R^1$ and $R^2$ independently of one another denote $C_1$-$C_{20}$-alkyl, preferably $C_1$-$C_7$-alkyl,
$R^3$ and $R^4$ independently of one another denote $C_1$-$C_4$-alkyl, preferably $C_1$-$C_2$-alkyl,
or the groups $R^1$ + $R^3$ and/or $R^2$ + $R^4$ together denote $-(CH_2)_4-$ or $-(CH_2)_5-$
and carrying out the reaction at temperatures between about 80 and 160 ° C.

**2.** A process as claimed in claim 1, wherein N,N-diisopropylamine, N,N-di-sec.-butylamine or N,N-dicyclohexylamine are used as secondary aliphatic amines having alkyl groups which are branched in the 1-position.

**3.** A process as claimed in either of claims 1 and 2, wherein, in the event that N,N-di-sec.-butylamine is used, the reaction is carried out without the use of an inert solvent.

**4.** A process as claimed in any of claims 1 to 3, wherein the reaction is carried out at temperatures between about 100 and 130 ° C.

## Revendications

**1.** Procédé pour préparer des chlorures de carbamoyles dérivant d'amines secondaires, par introduction de phosgène dans les amines secondaires en phase liquide préalablement mises en place, éventuellement dissoutes dans un solvant inerte, procédé caractérisé en ce qu'on utilise, comme amines secondaires, des amines aliphatiques secondaires qui contiennent des radicaux alkyles ramifiés à la position 1 et qui répondent à la formule I :

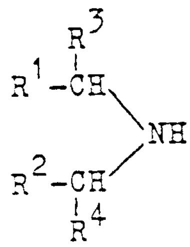 (I),

dans laquelle

$R^1$ et $R^2$ représentent chacun, indépendamment l'un de l'autre, un alkyle en $C_1$-$C_{20}$, de préférence en $C_1$-$C_7$,

$R^3$ et $R^4$ représentent chacun, indépendamment l'un de l'autre, un alkyle en $C_1$-$C_4$, de préférence en $C_1$ ou $C_2$, ou

$R^1$ et $R^3$, et/ou $R^2$ et $R^4$, représentent ensemble un radical $-(CH_2)_4-$ ou $-(CH_2)_5-$,

et en ce qu'on effectue la réaction à des températures comprises entre environ 80 et 160 °C.

**2.** Procédé selon la revendication 1 caractérisé en ce qu'on utilise, comme amines aliphatiques secondaires contenant des radicaux alkyles ramifiés à la position 1, la N,N-diisopropylamine, la N,N-di-sec-butylamine ou la N,N-di-cyclohexylamine.

**3.** Procédé selon l'une des revendications 1 et 2, caractérisé en ce que, dans le cas où on utilise la N,N-di-sec-butylamine, on opère sans solvant inerte.

**4.** Procédé selon l'une quelconque des revendications 1 à 3, caractérisé en ce qu'on effectue la réaction à des températures comprises entre environ 100 et 130 °C.